# EUROPEAN PATENT APPLICATION

(11) **EP 4 635 986 A1**
(43) Date of publication of application: **22.10.2025**
(21) Application number: 24386044.2
(22) Date of filing: 17.04.2024
(51) Int. Cl.: C07K 16/30, G01N 33/574, A61K 39/00

(54) **ANTI-EC PEPTIDE MONOCLONAL ANTIBODY**

(71) Applicant: National and Kapodistrian University of Athens, 10679 Athens (GR); Armakolas, Athanasios, 171 22 Nea Smyrni (GR); Koutsilieris, Michael, 145 69 Anoixi (GR); Filippou, Anastasios, 157 71 Zografou (GR)
(72) Inventor: Armakolas, Athanasios, 171 22 Nea Smyrni (GR); Koutsilieris, Michael, 145 69 Anoixi (GR); Filippou, Anastasios, 157 71 Zografou (GR)
(74) Representative: Roukounas, Dimitrios

(57) **Abstract**

A monoclonal antibody, or an antigen binding fragment thereof, that specifically binds to Ec peptide and the use of the antibody, or an antigen binding fragment thereof, in the treatment, diagnosis, or prognosis of cancer.

## Description

### Technical field of the invention

The present invention relates to monoclonal antibodies and uses thereof. More particularly, the present invention relates to anti Ec peptide (PEc) monoclonal antibodies and their use in the treatment, diagnosis, or prognosis of cancer.

### Background of the invention

The current treatment options for cancer have shifted more towards targeted therapies rather than traditional chemotherapy. Immunotherapy, using engineered antibodies or antibody fragments, directly targeting tumor cells or inducing anti-tumor immune responses, is now considered a main component of cancer therapy.

The role of IGF-1 in cancer is well established (1, 2). The *Igf-1* gene is transcribed and translated in humans in the form of multiple precursor IGF-1 polypeptides, namely isoforms. These are the IGF-1Ea the predominant form, IGF-1Eb and IGF-1Ec, all of which undergo postranslational modifications such as glycosylation and proteolytic cleavage, leading to the production of the mature IGF-1 (mlGF-1) (3, 4). Till recently the role of the carboxy- terminal E peptides (PEa, PEb and PEc) deriving from the cleavage of the pro- IGF -I isoforms, was not known (5, 6). The Ec peptide, the part of the IGF-1Ec isoform that is cleaved prior to the production of the mIGF-1, has been associated to prostate cancer progression.

The IGF system is gaining interest and has become one of the most explored areas in targeted anticancer therapy over the last decade since it is involved in several aspects of cancer development such as tumor growth, metastasis and invasion in a broad range of malignancies. Current therapeutic targeting of the IGF-1 signalling pathways focuses on blocking the IGF-1R or targeting the IGF-1R major ligand (IGF-1) (15-20). Targeting of the IGF-1 system apart from the obvious toxicity defects associated to its natural function (21-23) of the targeting of the IGF-1 system, seems to be problematic since none of these therapies was associated with significantly better overall survival in patients treated with chemotherapy and anti-IGF-1R or anti-IGF-1 vs patients treated with chemotherapy in various cancers. In the case of monoclonal antibodies against IGF-1R, it has been suggested that they can act as "biased" IGF-1R agonist to promote IGF-1R/b-arrestin1 association. This association results in ERK1/2 signalling pathway activation. Furthermore, these antibodies cannot neutralise the nuclear pool of the IGF-1R that possesses pro-tumorigenic activities. In the case of monoclonal antibodies against IGF-1 there are several Phase II and Phase III clinical trials.

Unlike IGF-1, the IGF-1Ec isoform is not normally expressed in healthy tissues. Its expression seems to be associated with tissue damage where its main function is Human Mesenchymal Stem Cells (HMSC) mobilization prior to repair (16-18). In cancer IGF-1Ec is only produced when cancer cells are challenged. In this case there is the production of the IGF-1Ea isoform (the main mature IGF-1 producing isoform) and the production of the IGF-1Ec isoform that increases as tumor progresses. The IGF-1Ec isoform is cleaved and it yields the mature IGF-1 and the PEc. Both molecules (IGF-1 and PEc) exert oncogenic actions through different receptors. Targeting of the IGF-1 or the IGF-1R covers a broad range of molecules involved in the IGF-1 system, including the IGF-1R and the heterohybrid receptors but it does not affect the action of PEc.

PEc secretion is central to tumor progression (proliferation, metastasis and repair). PEc overexpression in prostate cancer experimental models was associated with significant increase of cellular proliferation and induction of the epithelial to mesenchymal transition (EMT) process, which has a fundamental role in the mechanism of cancer cell invasion and metastasis. PEc effects are generated by activating the ERK 1/2 pathway, through an unknown receptor, other than IGF-1R, IR, or any of the IGF-1/IR hybrid receptors produced by the heterodimerization of the IR and IGF1R respective monomers (8, 9). The oncogenic role of PEc was also verified by the fact that the introduction of PEc overexpressing cells (normal and cancer cell lines) subcutaneously and orthotopically in SCID mice, leads to a significant elevation of both growth and metastatic rates of the resulting tumors compared to the tumors obtained from the wild type (wt) cell lines (9). Apart from its oncogenic effect, tumor secreted PEc was also found to be associated with host mesenchymal cells mobilization towards the tumor. The mobilisation of host MSC to the tumor has been associated to tumor repair (10).

Prostate cancer cell lines in *in vitro* conditions do not express the IGF-1Ec isoform. However, the tumors developed by the introduction of unmodified prostate cancer cells into SCID mice, expressed increasing amounts of IGF-1Ec as they progressed (10). Recent evidence suggests that the mechanism leading to tumor PEc secretion, is associated with the tumor-host immune cell-cell interaction (10). Immune cells secrete IL-6 upon prostate cancer cellular interaction. IL-6, through the JAK2/STAT3 pathway, seems to be a key molecule regulating tumor *igf-1Ec* and *il6* genes transcription. Tumor IL-6, acting in an autocrine/paracrine mode of action, further induces IGF-1Ec expression in prostate cancer, consequently leading to the elevation of the PEc amount and to tumor progression (10). Consistent with the laboratory evidence is the fact that IGF1-Ec expression determined in human prostate cancer biopsies was found to be directly positively associated to tumor grade and stage (11, 12).

Histopathology studies in cancer patients, using an anti-PEc polyclonal antibody, suggest that the presence of PEc is evident in various solid tumors including prostate, breast, endometrial, thyroid and colon cancer where is significantly associated to advanced stages (19-23).

### Summary of the invention

The present invention provides a monoclonal antibody, or an antigen binding fragment thereof, that binds specifically to Ec peptide (EPc).

A monoclonal antibody, or an antigen binding fragment thereof, according to the present invention comprises a heavy chain variable region comprising amino acid sequence SEQ ID NO: 2, or an amino acid sequence that is at least 90% identical to amino acid sequence SEQ ID NO: 2, and a light chain variable region comprising amino acid sequence SEQ ID NO: 3, or an amino acid sequence that is at least 90% identical to amino acid sequence SEQ ID NO: 3.

A monoclonal antibody, or an antigen binding fragment thereof, according to the present invention binds specifically to PEc and can be used in the diagnosis and/or prognosis and/or treatment of cancer.

The present invention provides also a pharmaceutical composition comprising a monoclonal antibody, or an antigen binding fragment thereof, as defined above and a pharmaceutically acceptable carrier.

The present invention provides also a monoclonal antibody, or an antigen binding fragment thereof, as defined above for use in the diagnosis and/or prognosis and/or treatment of cancer.

In addition, the present invention provides a method for the treatment of cancer comprising administering to a subject a monoclonal antibody, or an antigen binding fragment thereof, as defined above.

Furthermore, the present invention provides a method for the diagnosis and/or prognosis of cancer in a subject comprising contacting a sample obtained from the subject with a monoclonal antibody, or an antigen binding fragment thereof, as defined above.

### Brief description of the drawings

Figure 1 shows the results of the binding of an anti-PEc monoclonal antibody (MAb) according to the present invention to PEc.
Figure 2 shows the effect of different MAb concentrations on the ERK1/2 phosphorylation (a key molecule for proliferation and metastases/invasion).
Figure 3 shows in vitro effects of an anti-PEc MAb according to the present invention in prostate cancer cells with respect to proliferation, migration, invasion.
Figure 4 shows in vivo effects of an anti-PEc MAb according to the present invention in established tumors size.
Figure 5 shows in vivo effects of an anti-PEc MAb according to the present invention in established tumors on their molecular profile and on their size over prolonged treatment.
Figure 6 shows the correlation of the expression of PEc with human prostate tumors.
Figure 7 shows the correlation of the expression of PEc with human prostate tumors.

### Detailed description of the invention

The present invention provides a monoclonal antibody, or an antigen binding fragment thereof, that binds specifically to Ec peptide (PEc).

The anti-PEc monoclonal antibody, or an antigen binding fragment thereof, according to the present invention comprises
a) a heavy chain variable region comprising amino acid sequence SEQ ID NO: 2, or an amino acid sequence that is at least 90% identical to amino acid sequence SEQ ID NO: 2, and
b) a light chain variable region comprising amino acid sequence SEQ ID NO: 3, or an amino acid sequence that is at least 90% identical to amino acid sequence SEQ ID NO: 3.

Preferably, the monoclonal antibody, or an antigen binding fragment thereof, according to the present invention comprises
a) a heavy chain variable region comprising the amino acid sequence SEQ ID NO: 2, or an amino acid sequence that is at least 95% identical to amino acid sequence SEQ ID NO: 2, and
b) a light chain variable region comprising the amino acid sequence SEQ ID NO: 3, or an amino acid sequence that is at least 95% identical to amino acid sequence SEQ ID NO: 3.

More preferably, the monoclonal antibody, or an antigen binding fragment thereof, according to the present invention comprises
a) a heavy chain variable region comprising the amino acid sequence SEQ ID NO: 2, and
b) a light chain variable region comprising the amino acid sequence SEQ ID NO: 3.

The monoclonal antibody of the present invention, or an antigen binding fragment thereof, binds specifically to PEc and leads to a significant decrease in proliferation and metastatic rate of cancer cells that express PEc. Specifically, treatment of cancer cells that express PEc with the antibody of the present invention, or an antigen binding fragment thereof, leads to significant decrease of cellular proliferation and reversal of the cancer mesenchymal phenotype towards a more benign, epithelial phenotype, which explains the decrease in migration and invasion abilities of those cells. Therefore, the administration of the antibody of the present invention, or an antigen binding fragment thereof, to a subject inhibits tumor growth and metastasis.

The monoclonal antibody of the present invention, or an antigen binding fragment thereof, exhibits no toxicity towards non-cancerous cells.

An antibody according to the present invention includes an antibody of any class, such as IgG, IgA, or IgM.

Preferably, the monoclonal antibody of the present invention is an immunoglobulin G (IgG) antibody.

Preferably, the monoclonal antibody of the present invention is a humanized antibody.

The antibody of the present invention can be produced by following methods well known in the art. For example, it can be produced by first cloning in an expression vector, for example a mammalian expression vector, the DNA that corresponds to the amino acid sequences of the heavy chain and the light chain. The vector is then introduced into an expression system, such as a Chinese hamster ovary expression system, which produces the MAb.

An antigen binding fragment according to the present invention incudes for example, Fab, Fab', F(ab')2, Fd, Fv, and polypeptides that contain at least a portion of an immunoglobulin that is sufficient to confer specific antigen binding to the polypeptide.

The present invention provides also a pharmaceutical composition comprising a therapeutically effective amount of a monoclonal antibody, or an antigen binding fragment thereof, according to the present invention and a pharmaceutically acceptable excipient.

The composition may be formulated, for example, for oral, parenteral, intramuscular, intravenous, intraperitoneal, subcutaneous, or inhalation administration. The composition may have different forms, such as solid or liquid forms, for example it can have the form of solution, suspension, powder, emulsion, or capsule. Examples of pharmaceutically acceptable excipients, include solvents, buffering agents, emulsifying agents, preservatives, antioxidants, chelating agents, diluents, binders, disintegrants, or lubricating agents, which are well known in the art.

Examples of solvents, include water, saline, phosphate buffered saline, ethanol, isopropyl alcohol and mixtures thereof.

Examples of buffering agents include citric acid monohydrate, sodium citrate, sodium lactate, calcium lactate, acetic acid, sodium acetate, potassium acetate, dibasic potassium phosphate, monobasic potassium phosphate sodium hydrogen phosphate and sodium bicarbonate.

Examples of emulsifying agents include sodium lauryl sulfate, lecithin, polysorbate, sorbitan monooleate, polyethylene glycol, polypropylene glycol, polyvinyl alcohol, poloxamer nonionic surfactants, acacia, agar, alginic acid and sodium alginate.

Examples of preservatives include sodium benzoate, benzyl alcohol, benzalkonium chloride, benzethonium chloride, bronopol, cetrimide, ethanol, and parahydroxy benzoic acids and their alkyl esters.

Examples of antioxidants include sodium metabisulfite, butylated hydroxyanisole, butylated hydroxytoluene and ascorbic acid.

Examples of chelating agents include ethylenediaminetetraacetic acid and salts thereof (e.g., sodium edetate, disodium edetate, trisodium edetate, calcium disodium edetate, and the like), citric acid and salts thereof, fumaric acid and salts thereof, phosphoric acid and salts thereof, and tartaric acid and salts thereof.

Examples of diluents include lactose, sucrose, dextrose, mannitol sorbitol, inositol, calcium carbonate, sodium carbonate, calcium phosphate, dicalcium phosphate, microcrystalline cellulose, kaolin, sodium chloride, dry starch and cornstarch.

Examples of binders include hydroxypropylcellulose, hydroxyethylcellulose, carboxymethylcellulose, dextrose, xylitol, polyvinylpyrrolidone, polyethylene glycol, alginates and gelatin.

Examples of lubricating agents include magnesium stearate, calcium stearate, stearic acid, silica, talc, malt and sodium lauryl sulphate.

The present invention provides also a monoclonal antibody according to the present invention for use in the treatment of cancer in a subject.

Preferably, the subject is a human.

Preferably, the cancer is a solid cancer. More preferably, the cancer is selected from prostate cancer, breast cancer, colon cancer, bladder cancer, renal cancer, osteosarcoma, thyroid cancer, endometrial cancer, or neuroendocrine cancer. Even more preferably, the cancer is selected from prostate cancer, breast cancer, or colon cancer

The monoclonal antibody, or an antigen binding fragment thereof, is typically administered in the form of a pharmaceutical composition and it may be administered via a variety of routes including oral, intravenous, intramuscular, subcutaneous, transdermal, intraperitoneal, and topical route.

The monoclonal antibody, or an antigen binding fragment thereof, is generally administered daily, or more frequently, such as twice a day, or less frequently, such as every second day, or every week. The frequency of administration and the administered dosage depend on various factors, such as the type of cancer, the route of administration, the age of the subject, and the form of the administered composition, and can be determined by a person skilled in the art by using common general knowledge. For example, when the subject is a human, an effective dosage can be from 0.1 mg/kg to 80 mg/kg, preferably, from 1 mg/kg to 40 mg/kg, more preferably, from 6 mg/kg to 20 mg/kg

The present invention provides also an in vitro method for the diagnosis of cancer in a subject comprising contacting a sample obtained from the subject with a monoclonal antibody, or an antigen binding fragment thereof, according to the present invention. For example, a monoclonal antibody according to the present invention can be used in an ELISA assay for the detection of PEc in a sample, such as a blood sample, obtained from the subject. In another example, a monoclonal antibody according to the present invention can be used in an immunohistochemical assay for the detection of PEc in a sample of a tumor obtained from a subject.

The present invention provides also an in vitro method for the prognosis of cancer in a subject comprising contacting a sample obtained from the subject with a monoclonal antibody, or an antigen binding fragment thereof, according to the present invention. For example, a monoclonal antibody according to the present invention can be used in an ELISA assay for the detection of the presence of PEc in the a sample, such as a blood sample obtained from the subject, wherein elevated PEc levels are associated with more aggressive cancer phenotypes, Furthermore, a monoclonal antibody according to the present invention can be used in an ELISA assay performed before and during cancer treatment to evaluate the efficiency of the treatment, as well as after treatment to evaluate the efficacy of the treatment, wherein an increase of PEc in a sample obtained from the subject is associated with a cancer relapse. In another example, a monoclonal antibody according to the present invention can be used in an immunofluorescence assay, for the detection of circulating tumor cells (CTCs). CTCs expressing PEc peptide are associated with more agressive/metastatic cancer phenotypes.

### Examples

### Example 1

### Anti-PEc monoclonal antibody (Mab) production.

One hundred and twenty different monoclonal antibodies were produced using the hybridoma technique (Absea biotechnology ltd, Haidian district Beijin 100,085 China). Five mice were immunized with the human PEc or scrambled peptide (prior to the development of a valid isotype matching control antibody). Spleen cells were fused with immortal myeloma cells. The obtained monoclonal antibodies were tested for specificity and sensitivity against the antigen of interest (human PEc: YQPPSTNKNTKSQRRKGSTFEERK, SEQ ID NO:1) by an ELISA method. Different concentrations of each antibody ranged from 0 -1000 ng/ml were added in wells coated either with human Pec or with the scrambled peptide (1µg/ml). Ten different monoclonal antibodies were selected for their specificity and sensitivity against human (and mouse) PEc and they were further tested by dot blot analysis. The scramble peptide (CSKNKTQSREGTKFSEKYRPNTRPQ, SEQ ID NO:4) was used as a negative control. The most potent anti-PEc monoclonal antibody was selected. This Mab comprises a heavy chain variable region having the amino acid sequence SEQ ID NO: 2 and a light chain variable region having the amino acid sequence SEQ ID NO: 3 (Table 1).

**Table 1**

| **SEQ ID** | **Sequence** |
|---|---|
| SEQ ID NO: 2 | MGWTLVFLFLLSVTAGVHSEVQLQQSGAELVRSGASVKLSCAA SGFNLKDYYIHWVKQRPEQGLEWIGWIDPENGDTEYAPKFQGR ATMTADTSSNTAYLHLSSLTSEDTAVYYCFANDVDCWGQGTTL TVSSAKTTPPSVYPLAPGSAAQTNSMVTLGCLVKGYFPEPVTVT WNSGSLSSGVHTFPAVLESDLYTLSSSVTVPSSPRPSETVTCNV AHPASSTKVDKKIVPRDCGCKPCICTVPEVSSVFIFPPKPKDVLTI TLTPKVTCVVVDISKDDPEVQFSWFVDDVEVHTAQTQPREEQFN STFRSVSELPIMHQDWLNGKEFKCRVNSAAFPAPIEKTISKTKGR PKAPQVYTIPPPKEQMAKDKVSLTCMITDFFPEDITVEWQWNGQ PAENYKNTQPIMNTNGSYFVYSKLNVQKSNWEAGNTFTCSVLHE GLHNHHTEKSLSHSPGK |
| SEQ ID NO: 3 | MVSSAQFLGLLLLCFQGTRCDVVMTQPPLSLPVSLGDQASISCRS SQSLVHSNGKTYLHWYLQKPGQSPKLLIYKLSNRFSGVPDRFSG SGSGTDFTLKISRVEAEDLGVYFCFQSTHIPYTFGGGSKLEIRRAD AAPTVSIFPPSSEQLTSGGASVVCFLNNFYPKDINVKWKIDGSER QNGVLNSWTDQDSKDSTYSMSSTLTLTKDEYERHNSYTCEATHK TSTSPIVKSFNRNEC |

DNA coding for the amino acid sequence of this MAb was synthesized and cloned into the mammalian transient expression vector PETE V2 (Fusion Antibodies Pembroke Loop Road, Belfast Ireland). The MAb was expressed using CHO based transient expression system and the resulting antibody containing cell culture supernatants was clarified by centrifugation and filtration. MAb purification was obtained using AKTA affinity chromatography. Purified antibody was dialyzed into PBS. The purity of the MAb was determined to be >95% (reducing and denaturing SDS PAGE and size exclusion chromatography). MAb concentration was determined by measuring the absorbance at 280nm calculated using the standard extinction coefficient 205,000 M-1 cm-1 (or 1.0mg/mL = A280 of 1,37, assuming a MW = 150,000 Da). This MAb was used in the Examples hereinbelow.

### Example 2

### Binding analysis

Binding assays were performed by first capturing the IgG MAb of Example 1 by using an anti-mouse Fc Octet. The MAb capture biosensors were then submerged in wells containing 300nM of antigen. Steps were performed at 25°C at a constant flow rate of 1000 rpm. Dissociation rate constants (KD) were estimated using the Forte Bio data analysis software (Fusion Antibodies Pembroke Loop Road, Belfast Ireland). Due to the small size of the antigen (24aa) Octet binding analysis did not work. Therefore, binding analysis was carried out by a dot blot assay against different PEc fragments (aa: 1-12, 13-24, 1-6, 7-12, 13-19, 20-24. and against the whole PEc. Briefly, different concentrations of each fragment (10, 20, 40, 80 ng/mL) were applied onto a nitrocellulose strip, after drying the membrane was blocked by 5% BSA for 1 hour and then incubated with 2.5 µg/mL of the anti-PEc MAb for 1 hout at room temperature. After washing the membrane was incubated with an anti-mouse HRP, antibody (1:2,000 dilution); (Santa Cruz Biotechnology). Visualization took place by exposing the blots to x-ray film after incubation with freshly made ECL substrate for 3 min (SuperSignal, Pierce Biotechnology, Rockford, IL, USA). The results showed that the anti-PEc MAb is specific to the whole Ec peptide (24 amino acids) and to the last 12 amino acids and it does not attach efficiently to any of the smaller peptides.

### Example 3

### Competition assay

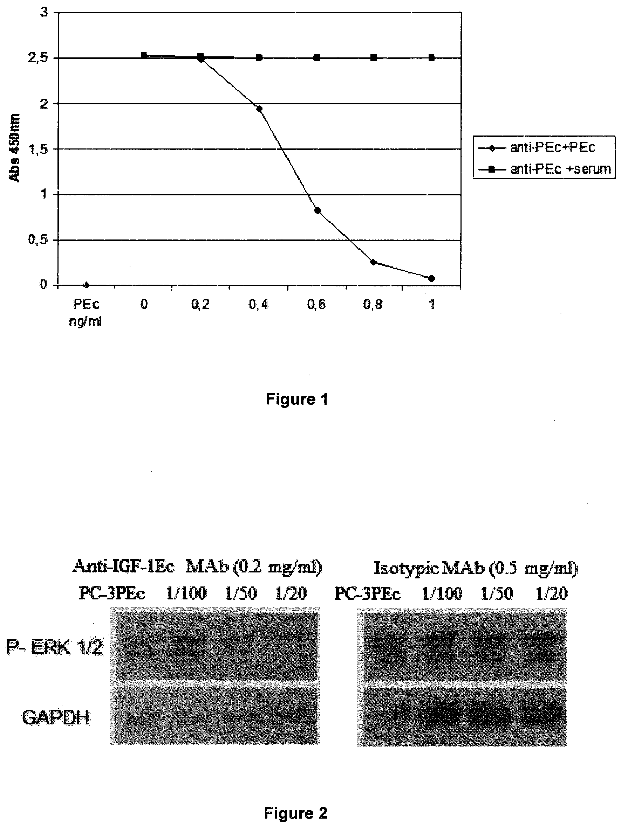

Wells of a microplate were coated with 1µg/ml of PEc. After washing, the anti-PEc MAb of Example 1 was introduced in each well (5 µg/ml) together with PEc at different concentrations/well (0.2, 0.4, 0.6, 0.8, 1, µg/ml). As a negative control human serum from 3 different individuals was used. Detection was carried out with a horseradish peroxidase conjugated goat anti-rabbit antibody at 1:1,000 dilution (Thermo Scientific [Thermo Fisher Scientific Inc., Waltham, MA, USA]). As a substrate, 3,3',5,5'-Tetramethylbenzidine (TMB) was used. The reaction stopped by administrating 2 mol/L sulphuric acid solution. The absorption of the plate was measured within 20 min at 450 nm and 540 nm as reference using a microplate reader (VersaMax, Molecular Devices, Sunnyvale, CA, USA). The results (Fig. 1) show that the MAb of the present invention exhibits specificity and sensitivity towards PEc. Introduction of different concentrations of PEc resulted in the reduction of absorbance (MAb attached on the PEc on the walls of the wells).

### Example 4

### In vitro effects of anti-PEc MAb in prostate cancer cell lines

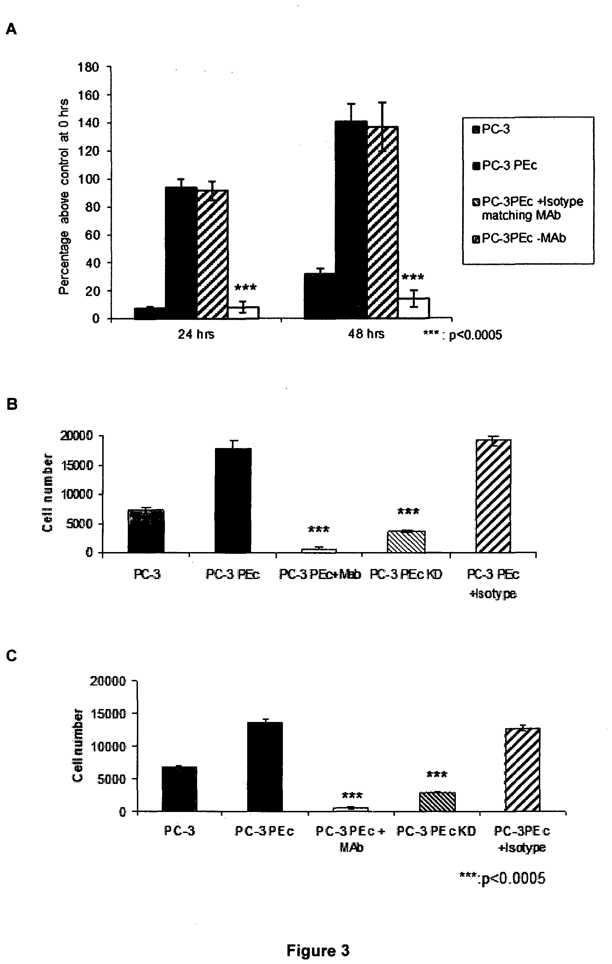

Prior to resemble this *in-vivo* characteristic of the prostate tumors, where PEc expression is gradually increased as tumor progresses, in *in-vitro* models, two prostate cancer cell lines (PC-3 androgen receptor (AR) positive and DU-145 AR negative) were modified to overexpress the PEc without affecting the mIGF-1evels. Recent evidence suggests that prostate cancer cell lines that overexpress the PEc, present elevated cellular proliferation levels, and a shift of their epithelial phenotype towards a mesenchymal, metastatic phenotype by activating ERK1/2 (7). Treatment of both modified cell lines with different concentrations of the anti-PEc MAb also leads to a gradual decrease of phosphorylated (activated) ERK 1/2 levels (Fig. 2). In prostate cancer, ERK1/2 activation apart from cellular proliferation, has been associated with the EMT and an increase in metastatic and invasive capacities (15).Treatment of both prostate cancer cell lines (PC-3PEc and DU145PEc) with the anti-PEc MAb of Example 1 lead to statistically significant decrease of the cellular proliferation (p<0.0005 in both cases) as measured at 24 and 48h (Fig.3a), in contrast to the unmodified prostate cancer cell lines, where treatment with anti-PEc did not significantly affect cellular proliferation. MAb treatment resulted in the significant reduction of migration (Fig. 3b) and invasion (Fig. 3c) capacities as was determined by transwell migration and invasion assays (p<0.0005, in both cases). Verification of the anti-PEc MAb effect on the migration (metastatic) ability of the modified prostate cancer cells was obtained by a wound healing assay, were the MAb treated cell lines presented a significantly lower migration rate (p<0.0005 at 18 hours for both cell lines). Isotype control: The monoclonal antibody generated by the scrambled peptide (a peptide possessing the same amino acids as the PEc but placed in a random order).

### Example 5

### In vivo effects of the anti-PEc MAb in Prostate cancer.

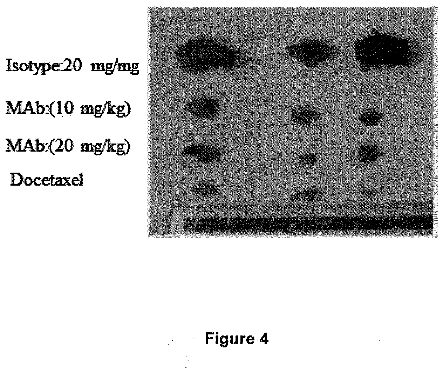

The *in vivo* effects of the anti-PEc MAb of Example 1 were also examined after subcutaneous (sc) inoculation of wild type (wt) unmodified prostate cancer cell lines (wtPC-3 and wtDU-145) into SCID mice (15). Both prostate cell lines used can generate tumors in 4 weeks after subcutaneous injection in SCID mice. In this study antibody treatment took place after the determination of palpable tumors in all the cases. Determination of the optimum MAb concentration for prostate cancer treatment was obtained by testing different concentrations of the anti-PEc MAb. The concentrations used were 5, 10 and 20 mg/kg, three times/week. Treatment of prostate tumours with different MAb concentrations resulted in significant tumour growth inhibition in 4 weeks, compared to the tumors treated with the isotype MAb (treatment with 20 mg/ml isotype control: tumor mass = 1.66g ± 0.3, with 0.5mg/ml anti-PEc MAb: tumor mass = 0.62g ± 0.1, with 10mg/ml anti-PEc MAb: tumor mass = 0.18g ± 0.04, with 20 mg/ml anti-PEc MAb: tumor mass = 0.16g ± 0.04, with Docetaxel: tumor mass = 0.137g ± 0.05. n=5, p<0.0005, in every case) (Fig. 4). Tumors treated with 10 and 20 mg/kg did not present significant difference in respect to their size and weight, suggesting that the optimum MAb concentration was the 10 mg/kg. Tumors treated with 10 mg/kg MAb for 4 weeks were also compared with the tumours treated with docetaxel for the same time period, with no significant difference in respect to their size and weight.

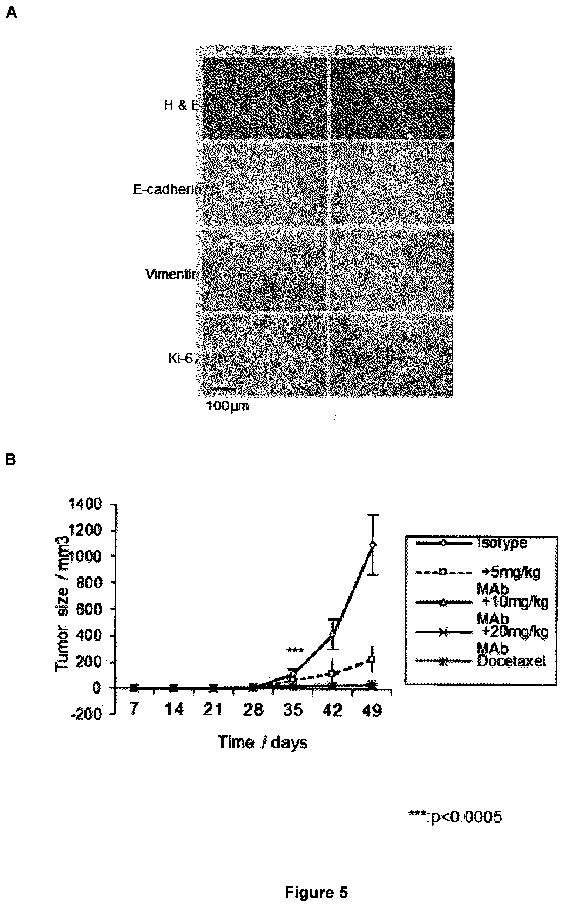

Immunohistochemical examination of the tumors documented that treatment with the anti-PEc MAb was associated with significant increase of the epithelial marker E-Cadherin, the reduction of the mesenchymal marker Vimentin and a decrease of the cellular proliferation marker ki-67 (Fig. 5a). These results were also verified by qRT PCR where the results obtained suggested statistically significant differences in Vimentin, E-Cadherin and Ki-67 expression (p<0.0005 in all the treated vs untreated subjects) indicating the efficiency of anti-PEc MAb in ceasing tumour progression.

In a different set of experiments, prostate tumours, resulting from subcutaneous (sc) inoculation of wtPC-3 and wtDU-145 cells into SCID mice, were treated with 10 mg/kg anti-PEc MAb three times / week, for a total of 8 weeks. Tumor volume was measured daily by calliper. Treatment for 5 weeks, led to significant tumor growth inhibition similar to that obtained by docetaxel (10 mg/kg, twice/week) and overall, to a statistically significant size difference when compared with the tumors treated with the isotype Mab control (for PC-3 cells: isotype control: 546 mm³ ± 57, anti-PEc MAb: 5mg/ml: 270mm³ ± 45, 10mg/ml: 20mm³ ± 4, 20mg/ml: 18mm³ ± 6, Docetaxel: 18mm3 ± 3, n=10 in every case, p<0.005 similar were the results for DU145 cells), (Fig. 5b). The tumor size difference between isotype treated tumors and anti-PEc MAb treated tumors further increased at 60 days (for PC-3 cells: isotype control: 1320 mm³ ± 212, anti-PEc MAb: 5mg/ml: 532 mm³ ± 105, 10mg/ml: 31mm³ ± 4, 20mg/ml: 26mm³ ± 6, Docetaxel: 28mm³ ± 3, n=10 in every case, p<0.0005 for every case for both cell lines). Tumors were also examined for the expression of P ERK1/2 (proliferation and EMT). Prostate tumors treated with the anti-PEc MAb presented a significant reduction of P ERK1/2 levels in comparison to the untreated tumours.

### Example 6

### Effects of the anti-PEc MAb of Example 1 in prostate cancer metastasis.

Epithelial to Mesenchymal Transition (EMT) is a central process to metastases, that normally occurs as tumors progresses as a response to harsh for the tumor conditions (space limitation, immune response of the host, hypoxia, NO2 species). The *in vivo* observation of the reversal of the cancer mesenchymal phenotype after the administration of the anti PEc MAb of Example 1, was further examined in the context of metastasis. Sentinel nodes as well as different tissues (liver, lungs, kidneys) were collected from SCID mice that developed prostate cancer and they were treated with anti-PEc MAb (n=20). Similarly, the same tissues were collected from untreated controls (n=10). Collection took place at 50 days after tumor establishment. In every case, tissues were stained (H&E) and examined by an experienced pathologist till paraffin block exhaustion.

None of the 20 mice treated with the anti-PEc MAb presented evidence of a possible sentinel node infiltration or of distal metastases. Six out of the 10 untreated mice presented evidence of sentinel node infiltrations, as was determined by imunohistochemical analysis evaluation and/or by significant size increase. Furthermore 3 out of the 6 mice presented distal metastases in the liver.

### Example 7

### PEc expression in prostate cancer patient biopsies

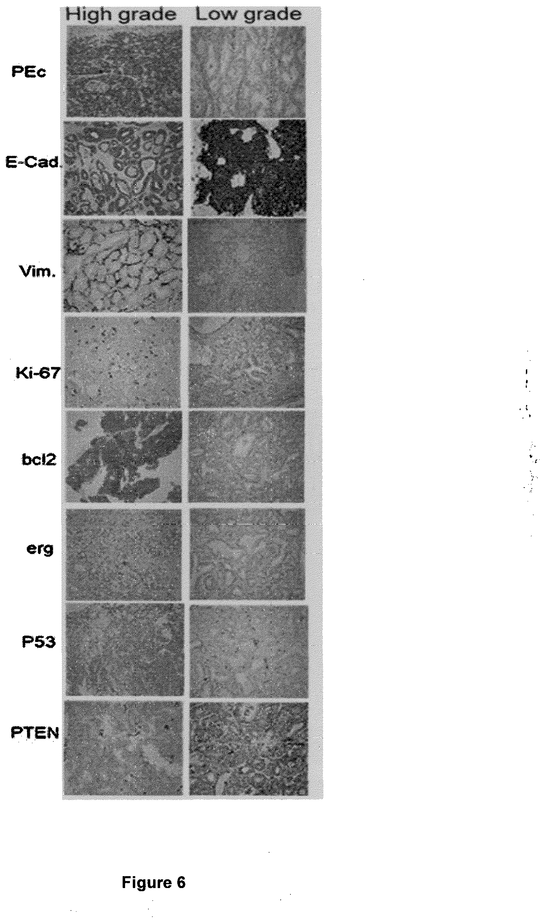

The levels of expression of IGF-1Ec were examined in 100 early stage (non-metastatic) prostate cancer patients (Fig. 6). Immunohistochemical expression of stained proteins was classified as either grade 1 (weak intensity), grade 2 (moderate intensity) or grade 3 (strong intensity). The H scoring system, incorporating both intensity and distribution of staining, was used for semi-quantitative analysis of protein immunoreactivity. More specifically, the percentage of positive cells was measured in every section and multiplied by 1, 2 and 3, respectively (grade 1 score= percentage with grade 1 expression x 1; grade 2 score= percentage with grade 2 expression x 2; grade 3 score = percentage grade 3 expression X 3). A total score between 0 and 300 was obtained for each case (total score = grade 1 score + grade 2 score + grade 3 score). In all the cases elevated IGF-1Ec was associated with, upregulation of vimentin and Ki-67 and a downregulation of E cadherin and PTEN protein (Fig. 6).

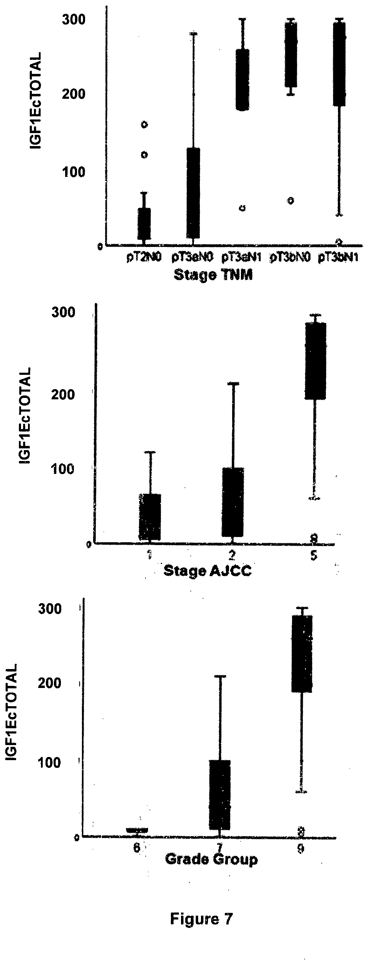

The IGF-1Ec h score was found significantly lower in prostate tumors in patients of stage T2N0 (AJCC) as compared to the tumours of patients of stage T3aN0 (p<0.0005). Patients of stage T3aN1 and T3bN0, presented significantly higher tumour IGF-1Ec levels compared to that of T3N0 patients (p<0.0005) (Fig. 7). All of our patients were of Gleason Score 6, 7 and 9. IGF-1Ec expression was significantly higher in the patients with gleason score 7 compared to those with Gleason score 6 (p<0.005). Patients with Gleason score 9 presented significantly higher levels of IGF-1Ec compared to those with Gleason score 7 (p<0.0005) (Fig. 7). Similarly, patients that presented extraprostatic extension were significantly associated with elevated PEc expression (p<0.0005).

Tumor cribriform architecture seems to be another factor that is associated to the IGF-1Ec expression levels. Higher cribriform grade was associated with significantly elevated levels of IGF-1Ec compared to tumors with lower cribriform (p<0.0005). Finally, elevated seminal vesicle infiltration presented significantly higher IGF-1Ec levels than prostate tumors that were not associated with invasive potential (p<0.0005 in both cases).

### Example 8

### Effect of the anti-PEc MAb of Example 1 on human prostate tumor xenografts

Approximately 0.7 cm³ of prostate tumors from 3 individuals with prostate cancer (all of which were of stage T3bN1M0 and high gleason score 8-9) were donated after total prostatectomies and tumor characterization. Informed consent was obtained in every case.

Human xenografts from prostate cancers were generated after sc inoculation of about 0.5 cm³ of tumor into SCID mice (n=3). The lump disappeared in 3 days and palpable tumors developed in 13 to 19 weeks (tumor 1 in 13 weeks, tumor 2 in 18 weeks, tumor 3 in 19 weeks). Tumors were allowed to grow up to 1.5 cm³ and they were then treated with 20 mg/ml anti PEc MAb of Example 1 intravenously for a period of 14 days during which mice were subjected to 4 injections. Starting point at time 0 was set as the point where tumor volume was about 1.5 cm³, the second injection was obtained at 48 hrs, the 3^{rd} at 100 hrs and the 4^{th} at 148 hrs. Treatment of SCID mice with anti PEc MAb resulted in a significant reduction in tumor volume in 76 hours (from 1.5 cm³ ±0.1 to 0.3 ± 0.02, p=0.0003). Tumor volume decrease continued over the whole period of monitoring but with lower rate. At day 14 (336 h) tumors were extracted and measured. Tumor volume in all the cases was about 0.1 cm³ as measured by calliper.

Analysis and comparison of the epithelial marker E-cadherin, the mesenchymal marker Vimentin and of the proliferation marker Ki67 of the human tumors before inoculation into SCID mice and after treatment with anti PEc MAb indicated significant reduction in the levels of Vimentin (p <0.0005 combined cases), a significant increase of E-Cadherin levels (p<0.0005 combined cases) and a significant decrease of Ki67 levels (p<0.0005 combined cases). The results obtained indicate a reduction in cellular proliferation and a switch of the cancer mesenchymal cells which are associated to metastases and invasion, towards a more "benign" epithelial phenotype. Sentinel nodes as well as livers and lungs were examined for metastases (IHC). None of these tissues exhibited evidence of infiltration.

### Example 9

### Antibody toxicity and tissue distribution

### -In vitro

The in vitro off target effects of the anti-PEc MAb of Example 1 were examined in two normal human cell lines, one prostate (HPrEc) and one fibroblast (HFL-1). In this case the normal cells were incubated with either, serum, peripheral blood mononuclear cells (PBMCs) or serum and PBMCs from 5 different individuals. After 24 hours the anti-PEc MAb was introduced for 12 hours and the non-cancerous cells were isolated from the PBMCs and analyzed with Anexin/PI, prior to determine the levels of apoptosis associated to the anti-PEc MAb. No significant differences were observed in respect to the induction of apoptosis after the administration of anti-PEc MAb in both normal cell lines used, nor when administration of the anti-PEc MAb was obtained in the presence of serum, PBMCs and serum +PBMCs.

In order to determine the effects of the anti-PEc MAb in normal cell survival over 48 h, non-cancerous cells were introduced with either, serum, PBMCs or serum and PBMCs from 5 healthy individuals for 24 hours followed by the introduction of the anti-PEc MAb. The non-cancerous prostate and fibroblast cells were isolated after 24 and 48 h and counted by trypan blue and MTT analysis. No statistically significant differences were observed in any of the comparisons between MAb treated and untreated cell lines, indicating that in *in vitro* conditions the anti-PEc MAb is not associated with any cytotoxic effects and this is evident due to the fact that non- cancerous cells produce almost negligible amounts of PEc even when challenged with factors of the immune system.

### -In vivo

The anti PEc MAb of Example 1 was examined for toxic side effects as well as for its distribution in mouse tissues. For the toxicity determination, 10 wt mice (n=10) were injected 4 times / week with 30 mg/kg anti PEc MAb for 60 days. Mice were assessed daily in respect to their appearance (body weight and coat condition), body function (dyspnoea and/or tachypnoea, food intake), environment (loose stools or diarrhoea, blood in diarrhoea) and behaviour (handling, aggression, abnormal gait, abnormal posture, reluctance to move), according to the Severity Assesment Framework of the European Commission.

None of the characteristics examined presented significant differences between treated and untreated mice. At the end of 60 days mice were sacrificed and tissues were collected stained and analysed in respect to tissue and cellular integrity. The tissues examined were lungs, liver, kidneys, heart and muscle. None of the tissues examined presented evidence of deterioration or structural alterations as assessed by an experienced pathologist.

### Tissue distribution

Tissue distribution of the MAb examined in this study was carried out using the biotinylated version of the anti-PEc Mab of Example 1. Five SCID mice (n=5) with already established tumors were injected with 20 mg/kg biotynilated MAb. After 24 h, mice were sacrificed and tissues were collected and analysed with an anti-biotin MAb. Tissues and tumors were then collected and examined for the presence of the anti-PEc MAb with an anti-biotin antibody (Abcam). The tissues examined were lung, liver, muscle, and kidney. It was determined that anti-PEc MAb was only present in the tumor and it was undetectable in all the healthy tissues examined.

### Example 10

### Alternative means of targeting the pathway

The IGF axis plays important roles in cancer progression and metastasis (15). The PEc arises from the proteolytic cleavage of the IGF-1Ec isoform. Recent evidence suggests that the PEc exerts its actions similarly to the IGF-1 through ERK1/2. anti-IGF-1R MAb and anti-IGF-1MAbs are in clinical trials for a number of different solid neoplasms. We therefore compared the effects of anti IGF-1R (10) and anti-IGF-1 (clone 7973) MAbs to those exerted by the anti-PEc MAb in prostate tumors. Subcutaneous prostate tumors were obtained by the introduction of PC-3 or DU145 cells in SCID mice. Tumors were treated for 4 weeks with 10 mg/kg of anti-IGF-1 MAb, anti-IGF-1R MAb and anti-PEc MAb. All the tumours extracted presented a significant size reduction compared to the tumors treated with the isotype control (Isotype treatment: 1.73g ± 0,19, anti-IGF-1R: 1.148g ± 0.082, anti-IGF-1: 1.07g ± 0.082, anti-PEc MAb: 0.062g ± 0.012, n=10 in every case, p<0.005). They also presented a significant increase in E cadherin expression (p<0.005) and a decrease in Vimentin and Ki-67expression (p<0.005 in both cases) as determined by qRT-PCR. Comparison of the tumours treated with the anti-IGF-1 and anti IGF-1R antibody (both from R&D Systems, Inc., MN, USA.) did not present significant difference in the aforementioned markers. Comparison of the tumors treated either with the anti IGF-1 Ab or the anti-IGF-1R antibody with the anti-PEc MAb indicated a significant difference in tumor size and in the expression of E cadherin and vimentin (p<0.0005 in every case). Sentinel nodes as well as livers and lungs were examined for metastases (IHC). None of these tissues exhibited evidence of infiltration. Toxicity: IGF-1R and IGF-1 MAb treated mice appeared to be lethargic and in 2 and 3 out of 10 respectively presented diarrhoea.

### Example 11

### The effect of anti-PEc MAb of Example 1 in breast and colon cancer

The effect of the anti-PEc MAb of Example 1 was examined in SCID mice that developed breast cancer after orthotopic inoculation of MCF-7 cells (n=20) and colon cancer after subcutaneous injection of DLD-1 cells (n=10), with similar results. In both cases the protocol for the antibody administration was kept the same as in prostate cancer. Therefore, after tumor establishment, 10 mg/kg were administrated into the SCID mice.

In breast cancer 2 out of 20 mice treated with the anti-PEc MAb retained palpable tumors compared to 10 out of 10 obtained in the untreated mice, 4 weeks after tumor detection. Due to the small size of the tumors obtained by the MAb treated mice we were not able to proceed with IHC. Therefore, we proceeded with qRT-PCR to examine the levels of E-Cadherin, Vimentin and Ki-67. Similarly, to the results observed in prostate cancer, breast tumors presented evidence of EMT reversal associated with a significant decrease of Vimentin and Ki-67 (p<0.0005 for both markers) and an increase of E-Cadherin compared to the untreated tumors (p<0.0001).
SCID mice with subcutaneous colon cancer tumors have been developed using wt DLD-1 cells. Treatment of palpable tumors with the anti-PEc MAb of Example 1 (4 weeks, 3 times/ week, 10 mg/kg) resulted in the development of significantly smaller tumors compared to the untreated tumors (Isotype MAb treatment: 2.36g ± 0.29, anti-PEc MAb treatment: 1.23g ± 0.16, Docetaxel treatment: 0.38g ± 0.06, n=10, p<0.0005). Treated tumors presented significant lower levels of Vimentin and Ki-67 expression and elevated levels of E cadherin as was observed by IHC and qRTPCR (p<0.0005 in every case). Western blot analysis also presented evidence of pERK 1/2 reduction compared to the untreated tumours. Tumours that are given raise by DLD-1 cells are very aggressive and they have high infiltrating capacity repair. What was observed in respect to tumor infiltration is the fact that DLD-1 tumours treated with the anti-PEc MAb did not infiltrate into the mouse body, compared to the untreated tumours where in all cases tumours infiltrated the ribcage and other tissues.

### References

1. Liu, G.; Zhu, M.; Zhang, M.; Pan, F. Emerging Role of IGF-1 in Prostate Cancer: A Promising Biomarker and Therapeutic Target. Cancers 15, 1287 (2023).
2. Brahmkhatri VP, Prasanna C, Atreya HS. Insulin-like growth factor system in cancer: novel targeted therapies. Biomed Res Int 2015, 538019 (2015).
3. Pierce JR, Martin BJ, Rarick KR, Alemany JA, Staab JS, Kraemer WJ, Hymer WC, Nindl BC. Growth Hormone and Insulin-like Growth Factor-I Molecular Weight Isoform Responses to Resistance Exercise Are Sex-Dependent. Front Endocrinol (Lausanne) 21(11), 571 (2020)
4. Ascenzi F, Barberi L, Dobrowolny G, Villa Nova Bacurau A, Nicoletti C, Rizzuto E, Rosenthal N, Scicchitano BM, Musarò A. Effects of IGF-1 isoforms on muscle growth and sarcopenia. Aging Cell 18(3), e12954 (2019).
5. Matheny RW, Jr., Nindl BC, Adamo ML. Minireview: Mechano-growth factor: a putative product of IGF-I gene expression involved in tissue repair and regeneration. Endocrinology 151(3), 865-875 (2010).
6. Schlegel W, Raimann A, Halbauer D, et al. Insulin-like growth factor I (IGF-1) Ec/Mechano Growth factor--a splice variant of IGF-1 within the growth plate. PLoS One 8(10), e76133 (2013).
7. Armakolas A, Philippou A, Panteleakou Z, Nezos A, Sourla A, Petraki C, Koutsilieris M. Preferential expression of IGF-1Ec (MGF) transcript in cancerous tissues of human prostate: evidence for a novel and autonomous growth factor activity of MGF E peptide in human prostate cancer cells. The Prostate 70(11), 1233-1242 (2010).
8. Armakolas A, Kaparelou M, Dimakakos A, Papageorgiou E, Armakolas N, Antonopoulos A, Petraki C, Lekarakou M, Lelovas P, Stathaki M, Psarros C, Donta I, Galanos PS, Msaouel P, Gorgoulis VG, Koutsilieris M. Oncogenic Role of the Ec Peptide of the IGF-1Ec Isoform in Prostate Cancer. Molecular medicine 21(1), 167-179 (2015).
9. Armakolas A, Dimakakos A, Loukogiannaki C, Armakolas N, Antonopoulos A, Florou C, Tsioli P, Papageorgiou E, Alexandrou TP, Stathaki M, Spinos D, Pektasides D, Patsouris E, Koutsilieris M. IL-6 is associated to IGF-1Ec upregulation and Ec peptide secretion, from prostate tumors. Mol Med 24, (2018).
10. Savvani A, Petraki C, Msaouel P, Diamanti E, Xoxakos I, Koutsilieris M. IGF-IEc expression is associated with advanced clinical and pathological stage of prostate cancer. Anticancer research 33(6), 2441-2445 (2013).
11. Li, H., Batth, I.S., Qu, X. et al. IGF-IR signaling in epithelial to mesenchymal transition and targeting IGF-IR therapy: overview and new insights. Mol Cancer 16, 6 (2017).
12. Osher E, Macaulay VM. Therapeutic Targeting of the IGF Axis. Cells 8(8), 895 (2019).
13. Gao J, Chesebrough JW, Cartlidge SA, et al. et al. Dual IGF-I/II-neutralizing antibody MEDI-573 potently inhibits IGF signaling and tumor growth. Cancer Res 71, 1029-1040 (2011).
14. Postel-Vinay S, Okuno S, Schuetze S, et al. et al. Safety, pharmacokinetics and preliminary activity of the anti-IGF-IR antibody CP-751,871 in patients with sarcoma. EJC Supplements 6, 122 (2008).
15. Rodon J, Patnaik A, Stein M, et al. et al. A phase I study of q3w R1507, a human monoclonal antibody IGF-1R antagonist in patients with advanced cancer. J Clin Oncol 25 (suppl), abstr 3590 (2007).
16. Kandalla PK, Goldspink G, Butler-Browne G, Mouly V. Mechano Growth Factor E peptide (MGF-E), derived from an isoform of IGF-1, activates human muscle progenitor cells and inducean increase in their fusion potential at different ages. Mechanisms of ageing and development 132(4), 154-162 (2011).
17. Hill M, Wernig A, Goldspink G. Muscle satellite (stem) cell activation during local tissue injury and repair. Journal of anatomy 203(1), 89-99 (2003).
18. Hill M, Goldspink G. Expression and splicing of the insulin-like growth factor gene in rodent muscle is associated with muscle satellite (stem) cell activation following local tissue damage. The Journal of physiology 549(Pt 2), 409-418 (2003).
19. Stavropoulos A, Varras M, Philippou A, et al. Immunohistochemical expression of insulin-like growth factor-1Ec in primary endometrial carcinoma: Association with PTEN, p53 and survivin expression. Oncol Lett 20(6), 395 (2020).
20. Karagiannis AK, Philippou A, Tseleni-Balafouta S, Zevolis E, Nakouti T, Tsopanomichalou-Gklotsou M, Psarras V, Koutsilieris M. IGF-IEc Expression Is Associated With Advanced Differentiated Thyroid Cancer. Anticancer Res 39(6), 2811-2819 (2019).
21. Christopoulos, P.F., Msaouel, P. & Koutsilieris, M. The role of the insulin-like growth factor-1 system in breast cancer. Mol Cancer 14, 43 (2015).
22. Alexandraki K. I., Philippou A., Boutzios G., Theohari E., Koutsilieris M., Kassiani Delladetsima I., Kaltsas G. A. IGF-IEc expression is increased in secondary compared to primary foci in neuroendocrine neoplasms. Oncotarget 8, 79003-79011 (2017).
23. Alagaratnam, S., Loizidou, M., Yang, S.y. et al. Increased expression of IGF-1Ec with increasing colonic polyp dysplasia and colorectal cancer. J Cancer Res Clin Oncol 146, 2861-2870 (2020).

## Claims

1. A monoclonal antibody, or an antigen binding fragment thereof, that binds specifically to peptide Ec, wherein the antibody, or an antigen binding fragment thereof, comprises
a) a heavy chain variable region comprising amino acid sequence SEQ ID NO: 2, or an amino acid sequence that is at least 90% identical to amino acid sequence SEQ ID NO: 2, and
b) a light chain variable region comprising amino acid sequence SEQ ID NO: 3, or an amino acid sequence that is at least 90% identical to amino acid sequence SEQ ID NO: 3.

2. The monoclonal antibody, or an antigen binding fragment thereof, according to claim 1, wherein the antibody, or an antigen binding fragment thereof, comprises
a) a heavy chain variable region comprising amino acid sequence SEQ ID NO: 2, or an amino acid sequence that is at least 95% identical to amino acid sequence SEQ ID NO: 2, and
b) a light chain variable region comprising amino acid sequence SEQ ID NO: 3, or an amino acid sequence that is at least 95% identical to amino acid sequence SEQ ID NO: 3.

3. The monoclonal antibody, or an antigen binding fragment thereof, according to claim 1 or 2, wherein the antibody, or an antigen binding fragment thereof, comprises
a) a heavy chain variable region comprising amino acid sequence SEQ ID NO: 2, and
b) a light chain variable region comprising amino acid sequence SEQ ID NO: 3.

4. The monoclonal antibody, or an antigen binding fragment thereof, according to any one of the preceding claims, wherein the antibody is a humanized antibody.

5. The monoclonal antibody, or an antigen binding fragment thereof, according to any one of the preceding claims, wherein the antibody is an IgG antibody.

6. The monoclonal antibody, or an antigen binding fragment thereof, according to any one of the preceding claims for use in the treatment of cancer in a subject.

7. The monoclonal antibody, or an antigen binding fragment thereof, for use according to claim 6, wherein the subject is a human.

8. The monoclonal antibody, or an antigen binding fragment thereof, for use according to claim 6 or 7, wherein the cancer is a solid cancer.

9. The monoclonal antibody, or an antigen binding fragment thereof, for use according to any one of claims 6 to 8, wherein the cancer is selected from prostate cancer, breast cancer, colon cancer, bladder cancer, renal cancer, osteosarcoma, thyroid cancer, endometrial cancer, or neuroendocrine cancer.

10. The monoclonal antibody, or an antigen binding fragment thereof, for use according to any one of claims 6 to 9, wherein the cancer is selected from prostate cancer, breast cancer, or colon cancer.

11. A pharmaceutical composition comprising a therapeutically effective amount of a monoclonal antibody, or an antigen binding fragment thereof, according to any one of claims 1 to 5 and a pharmaceutically acceptable excipient.

12. The pharmaceutical composition according to claim 11, wherein the composition is suitable for oral, parenteral, intramuscular, intravenous, intraperitoneal, subcutaneous, or inhalation administration.

13. A method for the diagnosis of cancer in a subject comprising contacting a sample obtained from a subject with the monoclonal antibody, or an antigen binding fragment thereof, according to any one of claims 1 to 5.

14. A method for the prognosis of cancer in a patient comprising contacting a sample obtained from a subject with the monoclonal antibody, or an antigen binding fragment thereof, according to any one of claims 1 to 5.
